(19) Europäisches Patentamt
European Patent Office
Office européen des brevets

(11) Veröffentlichungsnummer: **0 008 338**
**B1**

(12) # EUROPÄISCHE PATENTSCHRIFT

(45) Veröffentlichungstag der Patentschrift: **11.05.83**

(51) Int. Cl.³: **G 01 N 33/68,**
G 01 N 33/92

(21) Anmeldenummer: **79102204.9**

(22) Anmeldetag: **02.07.79**

(54) **Verfahren zur quantitativen Bestimmung eines Serumproteins in getrübten Serum- und Plasma-Proben und Mittel zur Durchführung des Verfahrens.**

(30) Priorität: **05.07.78 DE 2829531**

(43) Veröffentlichungstag der Anmeldung:
**05.03.80 Patentblatt 80/5**

(45) Bekanntmachung des Hinweises auf die Patenterteilung:
**11.05.83 Patentblatt 83/19**

(84) Benannte Vertragsstaaten:
**AT BE CH DE FR GB IT LU NL**

(56) Entgegenhaltungen:
**DE - A - 2 825 391**
**DE - B - 2 327 894**
**DE - B - 2 724 757**
**GB - A - 1 538 218**
**US - A - 3 260 648**
**US - A - 4 011 045**

(73) Patentinhaber: **Heuck, Claus-Christian, Dr. Dr.**
**Birkenweg 20**
**D-6901 Wilhelmsfeld (DE)**

(72) Erfinder: **Heuck, Claus-Christian, Dr. Dr.**
**Birkenweg 20**
**D-6901 Wilhelmsfeld (DE)**

(74) Vertreter: **Meyer-Dulheuer, Karl-Hermann, Dr. et al,**
**HOECHST Aktiengesellschaft Zentrale Patentabteilung Postfach 80 03 20**
**D-6230 Frankfurt/Main 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 008 338 B1

# Verfahren zur quantitativen Bestimmung eines Serumproteins in getrübten Serum- und Plasma-Proben und Mittel zur Durchführung des Verfahrens

Die Erfindung betrifft ein Verfahren zur quantitativen Bestimmung eines Serumproteins, insbesondere eines Apolipoproteins, in getrübten Serum- und Plasma-Proben, durch Trübungsmessung sowie ein Mittel zur Durchführung dieses Verfahrens.

Für die Bestimmung von Serumproteinen, insbesondere Apolipoproteinen sind automastische Verfahren einer Trübungsmessung bekannt. Sie haben sich jedoch nur für normolipämische Seren als geeignet erwiesen. Für hyperlipämische, insbesondere für hypertriglyeridämische Seren werden mit diesem Verfahren überhöhte Werte gemessen.

Für die Quantifizierung von Immunglobulinen und anderen Serumproteinen sind in neuerer Zeit Methoden mit Einsatz der Laser-Nephelometrie entwickelt worden. Hierbei hat sich insbesondere der Zusatz von Polyäthylenglykol (PEG) als günstig erwiesen. Charakteristischerweise wird in Gegenwart von PEG ein sogenanntes "Enhancement"-Phänomen durch Trübungsverstärkung beobachtet.

Nephelometrische Quantifizierung von Serumproteinen, insbesondere aber von Proteinanteilen an Serumlipoprotein werden durch die Eigentrübung in hyperlipämischen, d.h. triglycerid- und/oder cholesterinreichen Seren erschwert. Die Trübungsmessung einer immunoligischen Reaktion in nicht vorbehandelten Serumproben ergibt in diesen Fällen Meßergebnisse, die teilweise wesentlich über den Werten herkömmlicher Vergleichsverfahren liegen. Auch die Berücksichtigung der Eigentrübung eines Serums führt bei der Bestimmung von Apolipoproteinen zu keinem wesentlich besseren Ergebnis, da die Größe der Immunkomplexe aus mono-spezifischen Antikörpern und einem Apolipoprotein in intakten Lipoproteinen neben dem in den Lipoproteinen vorhandenen Apolipoproteinanteil ebenfalls vom dem Gehalt insbesondere an Triglycerid und Cholesterinestern in dem Lipoprotein abhängt und damit bei gleichem Apolipoproteingehalt zu einer unterschiedlichen Trübungsentwicklung führen kann. Eine Bestimmung von Apolipoproteinen durch eine quantitative Trübungsmessung von Immunpräzipitaten in Gegenwart von PEG hat sich ebenfalls als unpraktikabel erwiesen, da sich in diesem Falle eine starke unspezifische Trübung entwickelt.

Es sind zwei Wege bekannt, wie die durch Lipide verursachte Trübung vermindert werden kann:

1.) Durch Zusatz von trübungsaufhellenden Tensiden.
2.) Durch hydrolytischen Abbau von Lipiden in intakten Lipoproteinen.

Das Aufklaren von Serumproben durch Zusatz von nicht-ionischen Tensiden ist bereits beschrieben worden (Bergmeyer, H.U.: Enzymatische Analyse, 2. Auflage (1970) Band 1, S. 547—549 und 759—763). Insbesondere haben sich Fettsäureester oder Fettalkoholäther von Polyäthylenoxid als geeignet erwiesen (DE—B—2327894). Die Tenside werden hierbei in Konzentration von 0,2 bis 20 Vol% eingesetzt. Bei diesen Konzentrationen können zwar enzymatische Aktivitäten oder Metaboliten des menschlichen Köprers in Serumproben quantitativ ermittelt werden, hingegen werden nach weiteren Untersuchungsergebnissen immunologische Reaktionen zwischen einem Antikörper und einem Apolipoprotein in einem intakten Serumlipoprotein durch diese Tenside in Konzentration von 0,1 Vol% und darüber gehemmt. Dementsprechend ist die Übereinstimmung einer immunologischen Bestimmung von Apolipoproteinen in einen trüben Serum durch Trübungsmessung unter Bedingungen, die für Enzym- oder Metabolitmessungen ausreichen, mit einem der herkömmlichen Verfahren (z.B. radiale Immundiffusion) ebenfalls unzureichend.

Überraschend wurde gefunden, daß befriedigende Messungsergebnisse erzielt werden können, wenn man die Messung in Gegenwart eines Tensids der allgemeinen Formel

$$CH_3—(CH_2)_m—R—(CH_2—CH_2—O)_n—H \qquad (I)$$

durchführt in der R bedeutet:

$—O—$
$—NH—$
$—CH{=}CH—(CH_2)_x—NH—$
$—N(CH_2—CH_2—OH)—$

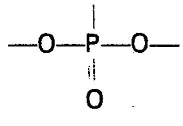

worin Y H— oder $CH_3—(CH_2)_q—$,
m und q, gleich oder verschieden, ganze Zahlen zwischen 6 und 26, vorzugsweise zwischen 6 und 18, n eine ganze Zahl zwischen 7 und 50, vorzugsweise zwischen 7 und 25 und x 7 oder 8 ist,
wobei das Tensid in einer Konzentration von $10^{-3}$ bis $10^{-1}$ Vol % eingesetzt wird.

Durch den enzymatischen Abbau von Lipiden in intakten Lipoproteinen kann ebenfalls die Eigentrübung eines lipämischen Serums verringert werden. Im Falle der Hydrolyse von Lipiden wird das Ausmaß der Trübungsentwicklung z.B. bei der immunologischen Reaktion zwischen Apolipoproteinen in Very Low Density Lipoproteinen (VLDL) und dem entsprechenden Antikörper im Vergleich zu einem unvorbehandelten Serum vermindert. Jedoch sind die Ergebnisse der quantitativen Bestimmung von Apolipoproteinen mit Hilfe der Laser-Nephelometrie nach einer enzymatischen Vorbehandlung von lipämischen Seren im Vergleich zur Methodik der radialen Immundiffusion oder der Immunelektrophorese nach Laurell unzureichend.

Alternativ wird erfindungsgemäß vorgeschlagen, daß man die Messung nach enzymatischem Abbau der Lipide und anschliessender immunologischer Reaktion mit dem entsprechenden Antikörper in Gegenwart eines Tensids der allgemeinen Formel (I), in der R, m und n die oben angegebene Bedeutung haben oder

$$—S—;$$

$$—\overset{\overset{\displaystyle O}{\|}}{C}—O—;$$

$$—CH{=}CH—(CH_2)_xO— \text{ oder}$$

$$—\underset{\underset{\displaystyle R'}{|}}{CH}—O— \text{ ist,}$$

wobei R' für $CH_3$ oder $C_2H_5$ steht, durchführt, wobei man das Tensid in einer Konzentration von $10^{-3}$ bis $10^{-1}$ Vol% einsetzt.

Vorzugsweise führt man die Messung mit Hilfe der Nephelometrie durch. Dadurch wird eine quantitative Bestimmung z.B. von Apolipoproteinen im menschlichen Serum auf eine schnellere, einfachere und preiswerte Weise ermöglicht, als sie mit den herkömmlichen Methoden erreicht werden konnte.

Die Erfindung erstreckt sich auch auf die Verwendung eines Tensids der allgemeinen Formel

$$CH_3—(CH_2)_m—R—(CH_2—CH_2—O)_n—H$$

in einer Konzentration von $10^{-3}$ bis $10^{-1}$ Vol% zur Serumaufbereitung für eine quantitative Bestimmung eines Apolipoproteins, wobei R, m und n die obengenannte Bedeutung haben.

Ein weiterer Erfindungsvorschlag betrifft ein Mittel zur Serumaufbereitung für die quantitative Bestimmung eines Apolipoproteins, das dadurch gekennzeichnet ist, daß es aus einer Mischung aus einem Tensid der allgemeinen Formel (I) und mindestens einem Lipid-Abbau-Enzym besteht, in der R, R', m und n die obengenannte Bedeutung haben.

Die Erfindung wird anhand der Zeichnungen näher erläutert. Es zeigt:

Fig. 1 Trübungsentwicklung durch Immunpräzipitate aus der Reaktion von Anti-Apolipoprotein B mit isolierten LDL und isolierten VLDL mit gleichem Gehalt an Apolipoprotein B.

Fig. 2 den Aufhellungseffekt der Antigen-Antikörperreaktion an isolierten VLDL bzw. LDL durch Zusatz eines Tensids;

Fig. 3 den Einfluß der Emulgatorkonzentration auf die Trübungsentwicklung einer immunologischen Reaktion zwischen Anti-Apolipoprotein B und Apolipoprotein B in intakten VLDL bzw. LDL;

Fig. 4 die Trübungsentwicklung der immunologischen Reaktion zwischen Anti-Apolipoprotein B und Apolipoprotein B im Serum nach Vorbehandlung mit verschiedenen Enzymen;

Fig. 5. einen Vergleich des erfindungsgemäßen Verfahrens mit dem der radialen Immundiffusion an normotriglyceridämischen Seren;

Fig. 6 einen Vergleich der beiden Verfahren an hypertriglyceridämischen Seren.

Die Laboratoriumdiagnostik der Fettstoffwechstörungen richtet sich nach den Empfehlungen von Fredrickson. Herkömmlicherweise werden hierfür die Konzentrationen von Cholesterin und Triglyceriden im Serum oder Plasma und in nach verschiedenen Verfahren fraktionierten Serumlipoproteinen gemessen. Die Untersuchungen gestatten jedoch nur eine symptomatische Aussage über die Art der Fettstoffwechselstörung. Aus neueren Untersuchungsergebnissen ist bekannt, daß Apolipoproteine, als Bestandteile der Lipoproteine des Serums, wichtige Funktionen in der Biochemie des Fettstoffwechsels erfüllen. Sie wirken teilweise als Aktivatoren, teilweise als Inhibitoren lipolytischer Enzyme. Für einige Apolipoproteine ist die physiologische Bedeutung nur zum teil aufgeklärt.

Von den neun bisher bekannten Apolipoproteinen, Apo-$A_1$, -$A_2$, -B, -$C_1$, -$C_2$, -$C_3$, -D, -E, -F, zeichnen sich das Apolipoprotein B dadurch aus, daß es den überwiegenden Proteinbestandteil (84—94%) in den Low Density Lipoproteinen (LDL), in denen der größte Anteil des Serumcholesterins transportiert wird, bildet. In den Very Low Density Lipoproteinen (VLDL) bestehen 40—50% des Proteinanteils aus Apolipoprotein B. Als ein integraler Teil dieser Lipoproteinklassen ist es ein Proteinkomplement zu zellständigen Rezeptoren, über die der Einstrom von Cholesterin in die Zelle reguliert und damit die intrazelluläre Synthese von Cholesterin unmittelbar beeinfluß wird. Im Hinblick auf die Entwicklung der Arteriosklerose kommt ihm daher eine besondere Bedeutung zu.

Aus diesem Grunde wird im Nachstehenden hauptsächlich die Bestimmung des Apolipoproteins B beschrieben. Es besteht jedoch kein Grund, das erfindungsgemäße Verfahrens nicht auch zur Bestimmung der anderen Apolipoproteine oder weiterer Serumproteine anzuwenden.

Die unten aufgeführte Tabelle zeigt die Korrelation der Bestimmung eines Apolipoproteins in menschlichen Serumproben mit Hilfe der Laser-Nephelometrie und mit Hilfe der radialen Immundiffusion. Die Volumenkonzentration des Tensids betrug $3\times10^{-3}$% Endkonzentration. Im Falle c betrug sie $3\times10^{-2}$%, im Falle d $6\times10^{-3}$%. In der letzteren Versuchsreihe wurde ein Tensid verwendet, bei dem R=—CH=CH—$(CH_2)_7$—NH— ist (Tensid A).

In anderen Versuchsreihen fand ein Tensid (Tensid B) Verwendung, bei dem

R=—CH—O—; R′=$C_2H_5$;
m=9; n=10 ist.

In der Tabelle ist:

N=Anzahl der untersuchten Serumproben
Tg=Triglyceridkonzentration (Grenzwerte in mg/dl)
Chol=Cholesterinkonzentration (Grenzwerte in mg/dl)
a=Steigung der Regressionsgeraden $y_{RID}=a.x_{naph}+b$
b=Schnittpunkt der Regressionsgeraden mit der y-Achse
r=Korrelation

| Ansatz | N | TG | Chol | a | b | r |
|---|---|---|---|---|---|---|
| a) +0,9% NaCl | 176 | 47—4300 | 47—4300 | 0,515 | +51,63 | 0,727 |
| b) +Tensid B ($3\times10^{-3}$%) | 176 | 47—4300 | 47—930 | 0,577 | +49,72 | 0,758 |
| c) +Tensid B ($3\times10^{-2}$%) | keine Standardisierung möglich | | | | | |
| d) +Tensid A ($6\times10^{-3}$%) | 203 | 60—2950 | 96—954 | 0,770 | +22,06 | 0,880 |
| e) +Triglyceridlipase (3 enzymatische Einheiten E) | 54 | 53—1535 | 133—725 | 0,250 | +83,42 | 0,559 |
| f) +Cholesterinesterase (200 mE) | 54 | 52—1535 | 133—725 | 0,491 | +63,86 | 0,613 |
| g) +Triglyceridlipase +Cholesterinesterase | 72 | 49—1200 | 96—800 | 0,804 | +26,57 | 0,820 |
| h) +Phospholipase D +Cholesterinesterase | 127 | 47—2700 | 42—1400 | 0,374 | +59,42 | 0,727 |
| i) Triglyceridlipase +Cholesterinesterase | 198 | 47—2700 | 42—1400 | 0,846 | +23,71 | 0,9262 |
| j) +Phospholipase D +Cholesterinesterase +Tensid B ($3\times10^{-3}$%) | 199 | 47—2700 | 42—1400 | 0,629 | +39,59 | 0,883 |
| k) Neuraminidase (60 mE) Cholesterinesterase (200 mE) Tensid B ($3\times10^{-3}$%) | 89 | 52—1790 | 100—450 | 1,423 | −3,76 | 0,905 |
| l) Neuraminidase (60 mE) Triglyceridlipase (3 E) Tensid B ($3\times10^{-3}$%) | 60 | 56—550 | 151—601 | 0,816 | +32,82 | 0,801 |

Der Tabelle ist zu entnehmen, daß ein Tensid des Typs Tensid A in der angegebenen Endkonzentration eine befriedigende Korrelation ergibt.

Bei Verwendung eines Tensids des Typs Tensid B vist zusätzlich ein enzymatischer Abbau der Lipide sinnvoll, wobei es sich zeigt, daß zu hohe Konzentrationen des Tensids eine Standardisierung unmöglich machen.

Folgende Enzyme haben sich für den enzymatischen Abbau von Lipiden bzw. Neuraminsäure als geeignet erwiesen;

| | | Aktivitätsbereich pro Ansatz |
|---|---|---|
| Triglyceridlipase | (EC. 3.1.1.3) | 0,5—10 E |
| Cholesterinesterase | (EC. 3.1.1.3) | 20—500 mE |
| Phospholipase D | (EC. 3.1.4.4.) | 10—100 mE |
| Neuraminidase | (EC. 3.2.1.18) | 10—150 mE |
| Phospholipase C | (EC. 3.1.4.3) | 5—500 mE |
| Carboxylesterase | (EC. 3.1.1.1) | 0,1—100 mE |

Hierbei hat sich gezeigt, daß eine Kombination verschiedener Enzyme (z.B. Triglyceridlipase und Cholesterinesterase) zu besseren Ergebnissen führt als die Verwendung eines einzigen Enzyms (z.B. Triglyceridlipase, Cholesterinesterase).

Trübungsmessungen sind Messungen von Streulichtintensitäten. Neben den apparativen Größen ist das Ausmaß einer Streulichtintensität von der Anzahl der in einer Lösung befindlichen Partikel, z.B. von Immunkomplexen, abhängig. Darüberhinaus wird die Streulichtintensität auch von dem Volumen der Partikel beeinflußt. Lipoproteine gehören mit zu den größten nicht zellulären sphärischen Bestandteilen des Serums. Mit einer Größe zwischen $10^3$ und $10^5$ nm verursachen sie auch ohne eine Komplexbildung mit einem Antikörper eine Eigentrübung des Serums.

Es war in Anbetracht der unterschiedlichen Größen der einzelnen Lipoproteine-Klassen zu erwarten, daß die Trübungsentwicklung durch Immunpräzipitate aus der Reaktion von Anti-Apolipoprotein B mit Apolipoprotein B in von einander isolierten LDL und VLDL unterschiedlich verlaufen müßte. Diesbezügliche Untersuchungen bestätigen vollauf das erwartete Phänomen (vgl. Fig. 1).

Der mit Hilfe der Immunelektrophorese nach Laurell gemessene Apolipoprotein-B-Gehalt betrug für die LDL 82 mg/dl, für die VLDL 40 mg/dl. Der Vergleich der Trübungsentwicklung bei 23°C für beide Lipoproteinklassen verdeutlicht, daß die Immunreaktion an den VLDL langsamer bis zum Erreichen des Trübungsmaximums verläuft, als die gleiche Reaktion an LDL. Zusätzlich ist die Trübungsentwicklung in VLDL trotz eines geringeren Apolipoprotein-B-Gehaltes stärker als in LDL.

Ferner wurde der Einfluß der Tenside auf die Antigen-Antikörperreaktion untersucht (Fig. 2).

Die einzelnen Lipoproteinfraktionen (Very Low Density Lipoproteine (VLDL) und Low Density Lipoproteine (LDL) wurden nach herkömmlichen Verfahren mit Hilfe der Ultrazentrifugation isoliert. Die Proben wurden entweder mit einer 0,9 g% NaCl-Lösung oder mit einer $10^{-2}$% Tensidlösung, bei der m=12 und n=9 ist, im Verhältnis 1+100 bei 23°C verdünnt.

Die Verdünnung wurde bei temperaturen zwischen 15°C und 54°C ausgeführt. Anschließend wurde 1 Teil der jeweiligen verdünnten Probe mit 2 Teilen einer Apo-B-Antikörperlösung, die mit einer physiologischen Kochsalzlösung im Verhältnis (1+5) verdünnt worden war, gemischt. Die Trübungsentwicklung wurde über einen Zeitraum von 180 Minuten mit Hilfe eines Laser-Nephelometers bei 23°C verfolgt.

Der Vergleich (Fig. 2) der Untersuchungen an VLDL und an LDL verdeutlicht, daß das Ausmaß der sich entwickelnden Trübung in Gegenwart des Tensids gegenüber dem gleichen Versuch, bei dem eine Verdünnung mit einer physiologischen Kochsalzlösung durchgeführt wurde, ausschließlich in der VLDL-Fraktion vermindert ist. Hingegen wird ein minimaler Einfluß des Tensids bei der gleichen Reaktion an den LDL festgestellt. In Gegenwart des Tensids wird das Trübungsmaximum bei der Reaktion an VLDL zu einem früheren Zeitpunkt erreicht als bei dem entsprechenden Versuch mit physiologischer Kochsalzverdünnung.

Des weiteren wurden Lipoproteine mit einer Kochsalzlösung oder einer Tensidlösung von unterschiedlicher Volumen-Endkonzentration ($3 \times 10^{-1}$%; $3 \times 10^{-2}$%; $3 \times 10^{-3}$%) im Verhältnis 1+100 verdünnt (Fig. 3). Die Mischung der Antikörperlösung mit den verdünnten Proben wurde in gleichem Verhältnis wie oben angegeben ausgeführt. Der Vergleich der bei der Antigen-Antikörperreaktion sich entwickelnden Trübung bestätigte, daß das verwendete Tensid vom Typ m=9, R'=—$C_2H_5$, n=10 in höheren Konzentrationen (0,2 Vol%) die Immunreaktion hemmt.

Fig. zeigt eine Untersuchung eines hyperlipämischen Serums (Triglyceridgehalt: 840 mg/dl, Cholesteringehalt: 330 mg/dl). Das Serum wurde mit physiologischer Kochsalzlösung im Verhältnis 1+100 verdünnt. Ein Aliquot von 300 $\mu$l wurde mit 20 $\mu$l einer Enzymlösung entweder aus 3 E. Triglyceridlipase (T) und 200 m.E. Cholesterinesterase (CE) oder aus 50 m.E. Phospholipase D (D) und 200 m.E. Cholesterinesterase (CE) über 30 Minuten bei 23°C vorinkubiert. (Alternativ können die Enzyme in der Tensidlösung gelöst zugegeben werden.) Danach wurde eine Antikörperlösung in dem

oben erwähnten Verhältnis zugesetzt und die sich entwickelnde Trübung über einen Zeitraum von 4 Stunden verfolgt.

Der Vergleich der Trübungsentwicklung nach Vorbehandlung mit verschiedenen Lipasen bestätigt eine Abnahme der Trübungsentwicklung. Diese Abnahme kann als ursächlich mit der Wirkung der Lipase, nämlich dem hydrolytischen Abbau von Lipiden in den Lipoprotein angenommen werden. Der kinetische Verlauf bestätigt ferner, daß das Maximum der Trübungsentwicklung nach Vorbehandlung mit den Lipasen früher erreicht wird, als bei einer unvorbehandelten Serumprobe.

Die Trübungsentwicklung kann quantitativ nephelometrisch gemessen werden. Vergleichsuntersuchungen an Seren mit einer Hyperlipämie, deren Gehalt an Apolipoprotein B mit dem in folgenden dargelegten Verfahren bestimmt wurde, zeigen eine hohe Übereinstimmung mit den Bestimmungswerten nach anderen Verfahren (radiale Immundiffusion, Immunelektrophorese nach Laurell).

Die Serumprobe wurden im Verhältnis 1+100 mit einer physiologischen Kochsalzlösung oder mit einer 0,01% Tensidlösung des Typs m=9, n=10, $R'=C_2H_5$ verdünnt und 30 Minuten lang mit den Enzymkombinationen vorinkubiert. Anschließend wurden die vorbehandelten Proben mit einem Anti-Apolipoprotein B-Serum in oben beschriebener Weise aufgearbeitet. Sämtliche Reaktionen wurden bei 23°C durchgeführt. Das Ablesen des Trübungsendwertes erfolgte 2 Stunden nach Zugabe des Antikörpers.

Der Vergleich der Untersuchungsergebnisse zeigt, daß weder der alleinige Zusatz eines solchen Tensids, noch eine ausschließliche Vorinkubation der Serumsproben mit lipolytischen Enzymen Meßwerte ergibt, die mit den aus der radialen Immundiffusion erhaltenen Daten in einem für labordiagnostische Zwecke aurreichendem Maße übereinstimmen. Ein kombiniertes Verfahren, in dem sowohl ein Tensid zugesetzt wird als auch eine Vorinkubation mit lipolytischen Enzymen zum Abbau der in Lipoproteinen vorhandenen Lipiden ausgeführt wird, ergibt hingegen eine Übereinstimmung der Meßwerte von mehr als 90% mit dem Verfahren der radialen Immundiffusion über den gesamten Bereich der im Serum auftretenden Triglyceridkonzentrationen bis zu 2700 mg/dl und Cholesterinkonzentrationen bis zu 1400 mg/dl.

Ein differenzierter Vergleich der Apolipoprotein B-Bestimmung aus Seren von Personen mit einer Normo- und Hypertriglyceridämie wurde mit Hilfe der radialen Immundiffusion und mit Hilfe des Lasernephelometrischen Verfahrens vorgenommen.

In Fig. 5 ist ein Vergleich der beiden Verfahren an normotriglyceridämischen Seren dargestellt (Normolipämie und Hyperlipoproteiämie vom Typ IIa).

Seren mit Cholesterinkonzentrationen <280 mg/dl: O

Seren mit Cholesterinkonzentrationen >280 mg/dl: △

Es ergibt sich die Korrelationsgerade $Y_{RID}=0{,}875\ X_{neph}+22{,}5$.

In Fig. 6 ist ein Vergleich der beiden Verfahren an hypertriglyceridämischen Seren dargestellt (Hyperlipoproteinämie vom Typ IV, V und IIb).

Seren mit Cholesterinkonzentrationen <280 mg/dl: O

Seren mit Cholesterinkonzentrationen >280 mg/dl: △

Die Korrelationsgerade ist hier $Y_{RID}=0{,}823\ X_{neph}+24{,}7$.

**Patentansprüche**

1. Verfahren zur quantitativen Bestimmung eines Serumproteins insbesondere Apolipoproteins in getrübten Serum- oder Plasmaproben durch Trübungsmessung nach Durchführung einer immunologischen Reaktion mit dem entsprechenden Antikörper, daddruch gekennzeichnet, daß man die Messung in Gegenwart eines Tensids der Allgemeinen Formel

$$CH_3—(CH_2)_m—R—(CH_2—CH_2—O)_n—H \qquad (I)$$

durchführt, in der R bedeutet:

—O—
—NH—
$—CH=CH—(CH_2)_x—NH—$
$—N(CH_2—CH_2—OH)—$

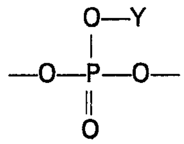

worin Y H— oder $CH_3—(CH_2)_q—$,
m und q gleich oder verschieden, ganze Zahlen zwischen 6 und 26, vorzugsweise zwischen 6 und 18, n eine ganze Zahl zwischen 7 und 50, vorzugsweise zwischen 7 und 25 und x 7 oder 8 ist,
wobei das Tensid in einer Konzentration von $10^{-3}$ bis $10^{-1}$ Vol % eingesetzt wird.

2. Verfahren zur quantitativen Bestimmung eines Apolipoproteins in getrübten Serum- oder Plasmaproben durch Trübungsmessung nach enzymatischem Abbau der Lipide und anschliessender immunologischer Reaktion mit dem entsprechenden Antikörper dadurch gekennzeichnet, daß man die Messung in Gegenwart eines Tensids der in Anspruch 1 angegebenen allgemeinen Formel (I), in der R, m, n und x die in Anspruch 1 angegebenen Bedeutungen haben oder R gleich

$-S-$;

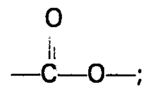

$-CH=CH-(CH_2)_x-O-$ oder

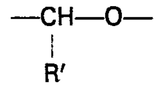 ist,

wobei R' für $CH_3$ oder $C_2H_5$ steht, durchführt, wobei man das Tensid in einer Konzentration von $10^{-3}$ bis $10^{-1}$ Vol % einsetzt.

3. Verfahren nach Anspruch 1 oder 2, dadurch gekennzeichnet, daß man die Messung mit Hilfe der Nephelometrie durchführt.

4. Mittel zur Serumaufbereitung für die quantitative Bestimmung eines Apolipoproteins, dadurch gekennzeichnet, daß es aus einer Mischung aus mindestens einem Lipid-Abbau-Enzym und einem Tensid der in Anspruch 1 angegebenen allgemeinen Formel (I) besteht, in der R, R', m, n und x die obengenannte Bedeutung haben.

**Revendications**

1. Procédé pour la détermination quantitative d'une protéine sérique, en particulier d'une apolipoproteine, dans des échantillons troubles de sérum ou de plasma, par la mesure du trouble après application d'une réaction immunologique avec l'anticorps correspondant, caractérisé en ce qu'on effectue la mesure en présence d'un agent tensioactif de formule générale

$$CH_3-(CH_2)_m-R-(CH_2-CH_2-O)_n-H \quad (I)$$

dans laquelle R représente

$-O-$
$-NH-$
$-CH=CH-(CH_{2x}-NH-$
$-N(CH_2-CH_2-OH)-$

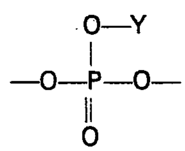

où Y est H ou $CH_3-(CH_2)_q-$,

m et q sont egaux ou différents et sont des nombres entiers entre 6 et 26, de préférence entre 6 et 18, n est un nombre entier entre 7 et 50, de préférence entre 7 et 25 et x est 7 ou 8,

l'agent tensioactif étant utilisé en une concentration de $10^{-3}$ à $10^{-1}$% en volume.

2. Procédé pour la détermination quantitative d'une apolipoprotéine dans des échantillons troubles de sérum ou de plasma, par mesure du trouble après une dégradation enzymatique des lipides et ensuite une réaction immunologique avec l'anticorps correspondant, caractérisé en ce qu'on effectue la mesure en présence d'un agent tensioactif de formule générale (I) selon la revendication 1, dans laquelle R, n et x ont les significations indiquées dans la revendication 1 où R représente également

$-S-$;

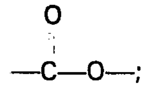

$—CH{=}CH—(CH_2)_x—O—$ ou

$—CH—O—$
$\quad|$
$\quad R'$

où R' représente $CH_3$ ou $C_2H_5$, l'agent tensioactif étant utilisé en une concentration de $10^{-3}$ à $10^{-1}$% en volume.

3. Procédé selon la revendication 1 ou 2, caractérisé en ce qu'on effectue la mesure à l'aide de la néphélométrie.

4. Réactif pour le traitement d'un sérum en vue de la dètermination quantitative d'une apolipoprotéine, caractérisé en ce qu'il consiste en un mélange d'au moins une enzyme dégradant un lipide et d'un agent tensioactif de formule générale I suivant la revendication 1, dans laquelle R, m et n ont les significations indiquées ci-dessus.

## Claims

1. A process for the quantitative determination of a serum protein, especially apolipoprotein, in turbid serum or plasma samples by nephelometry after having carried out an immunologic reaction with the corresponding antibody, which comprises measuring in the presence of a tenside of the formula

$$CH_3—(CH_2)_m—R—(CH_2—CH_2—O)_n—H \qquad (I)$$

in which R is

$—O—;$
$—NH—;$
$—CH{=}CH—(CH_2)_x—NH—;$
$—N(CH_2—CH_2—OH)—;$

$\quad\quad O—Y$
$\quad\quad |$
$—O—P—O—;$
$\quad\quad \|$
$\quad\quad O$

Y is $H—$ or $CH_3—(CH_2)_q—$;
*m* and *q*, being identical or different, each are an integer of from 6 to 26, preferably 6 to 18;
*n* is an integer of from 7 to 50, preferably 7 to 25; and
*x* is 7 or 8;
the tenside being used in a concentration of from $10^{-3}$ to $10^{-1}$% by volume.

2. A process for the quantitative determination of an apolipoprotein in turbid serum or plasma samples by nephelometry after enzymatic degradation of the lipids and subsequent immunologic reaction with the corresponding antibody, which comprises measuring in the presence of a tenside of the formula (I) in claim 1, in which R, m, n and x are as defined in claim 1 or in which R is

$—S—;$

$\quad O$
$\quad \|$
$—C—O—;$

$—CH{=}CH—(CH_2)_x—O—$ or

$CH—O—,$
$|$
$R'$

R' being $CH_3$ or $C_2H_5$; the tenside being used in a concentration of from $10^{-3}$ to $10^{-1}$% by volume.

3. The process as claimed in claim 1 or 2, which comprises measuring by means of nephelometry.

4. Agent for serum work-up for quantitative determination of an apolipoprotein, which consists of a mixture of at least one lipid-degrading enzyme and a tenside of the formula (I) in claim 1, in which R, R', m, n and x are as defined above.

FIG. 1

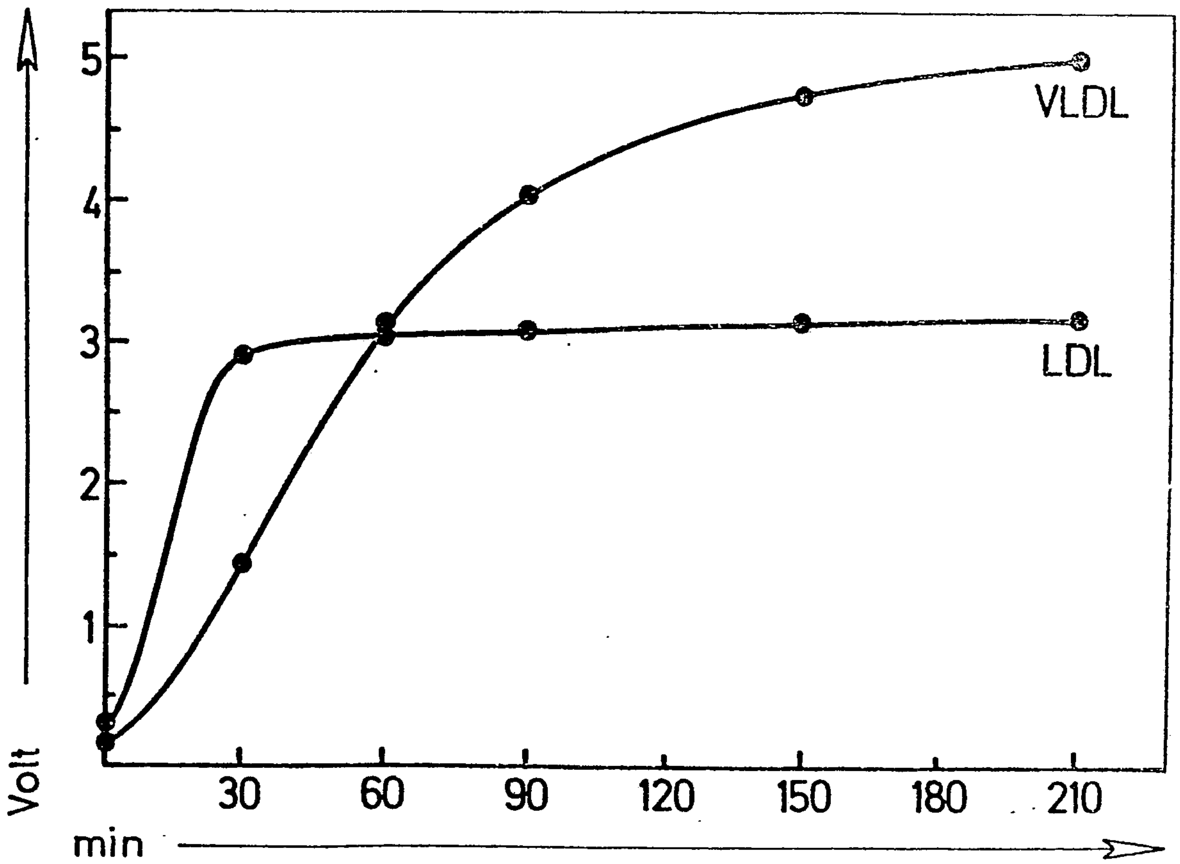

FIG. 2

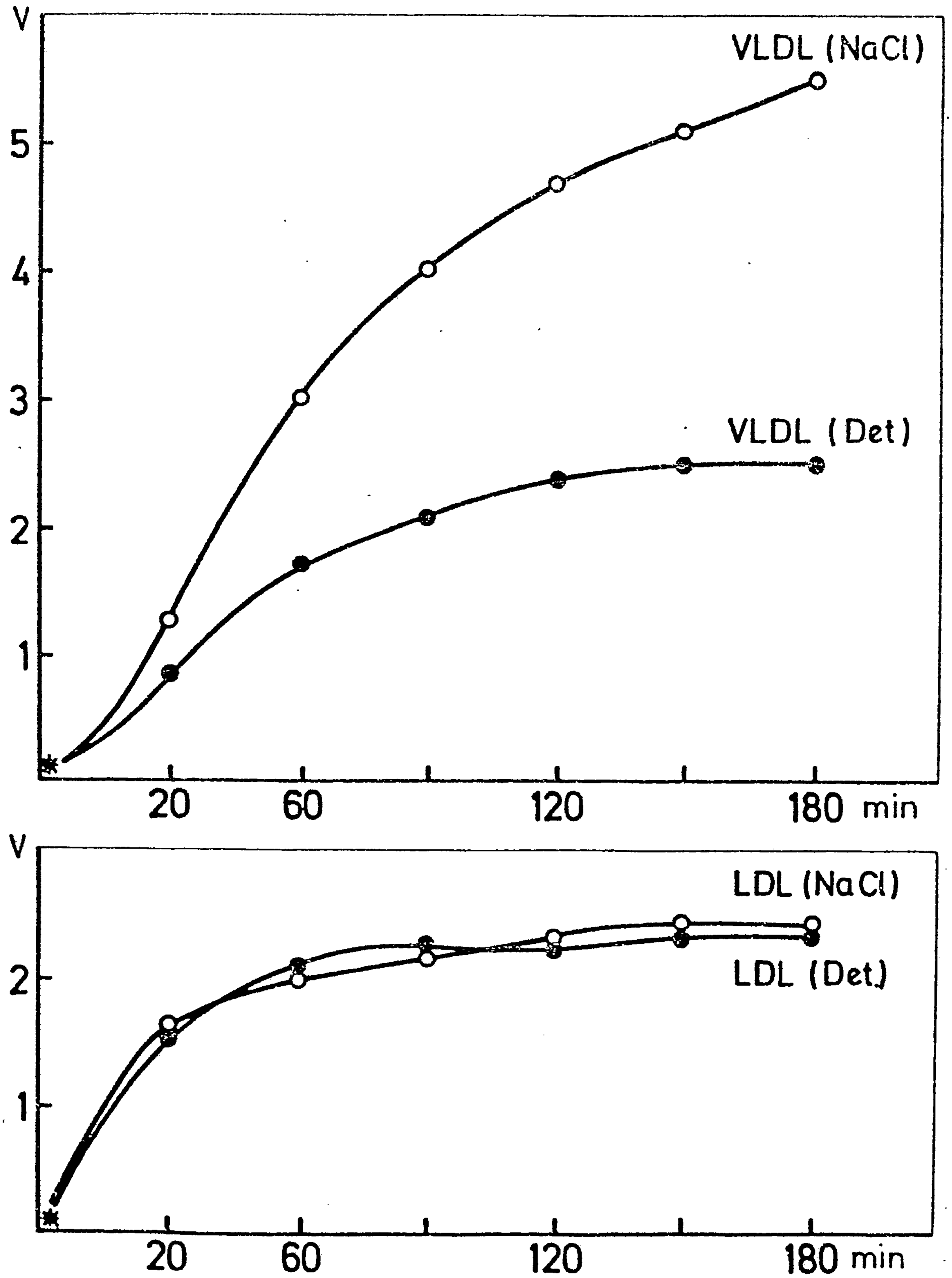
V
5
4
3
2
1
VLDL (NaCl)
VLDL (Det)
20
60
120
180 min
V
2
1
LDL (NaCl)
LDL (Det.)
20
60
120
180 min

FIG. 3

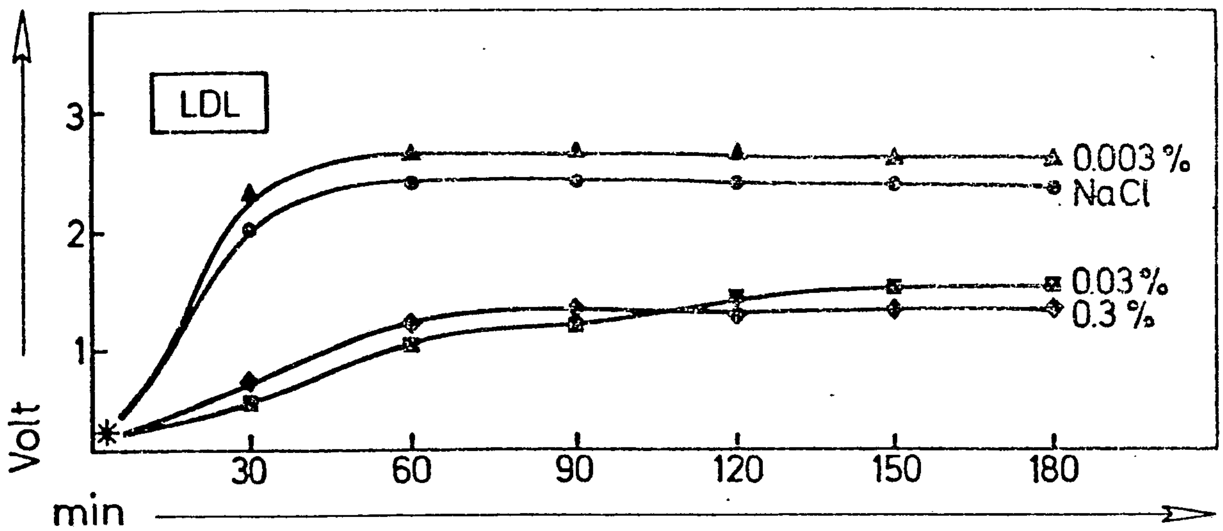
LDL
Volt
3
2
1
30
60
90
120
150
180
min
0.003 %
NaCl
0.03 %
0.3 %

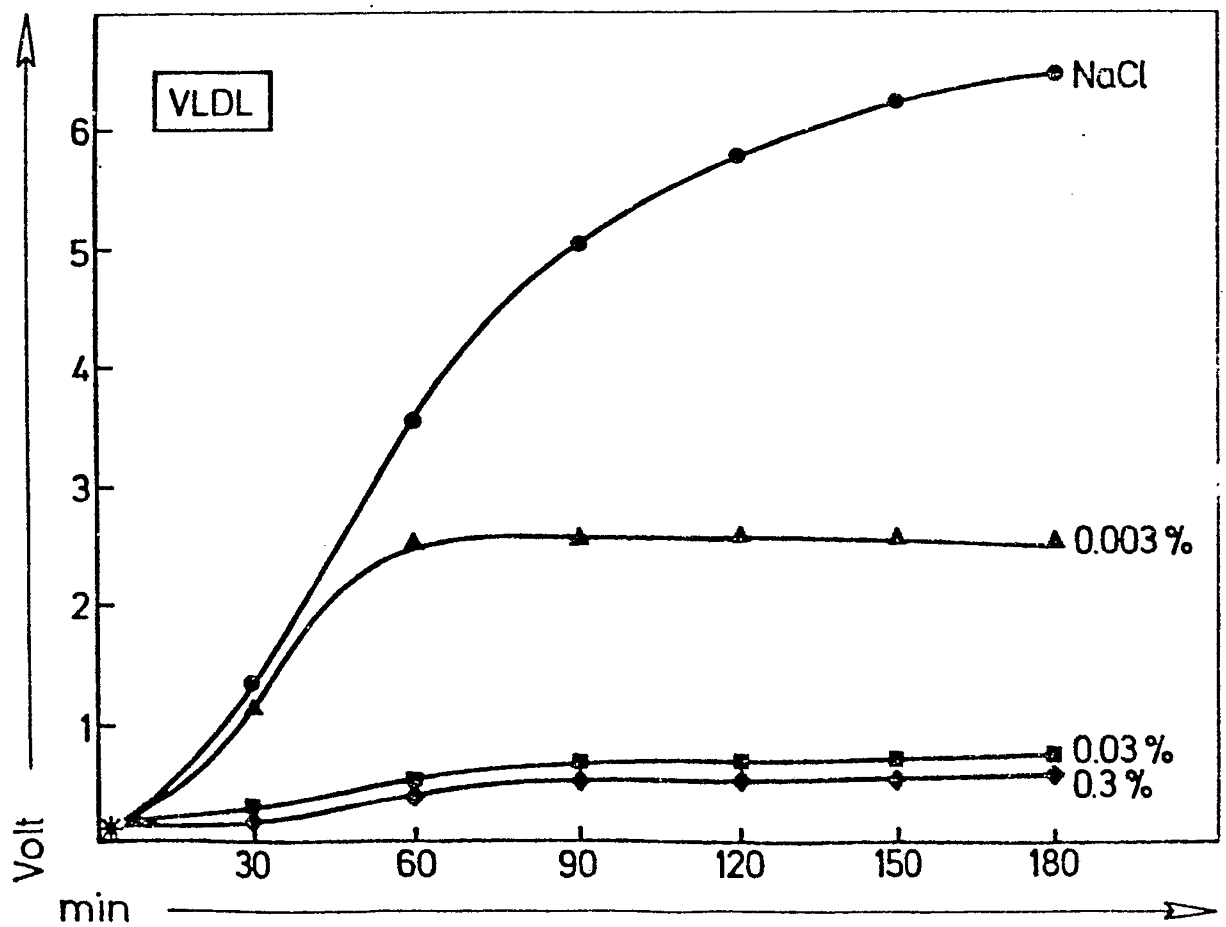
VLDL
Volt
6
5
4
3
2
1
30
60
90
120
150
180
min
NaCl
0.003 %
0.03 %
0.3 %

FIG. 4

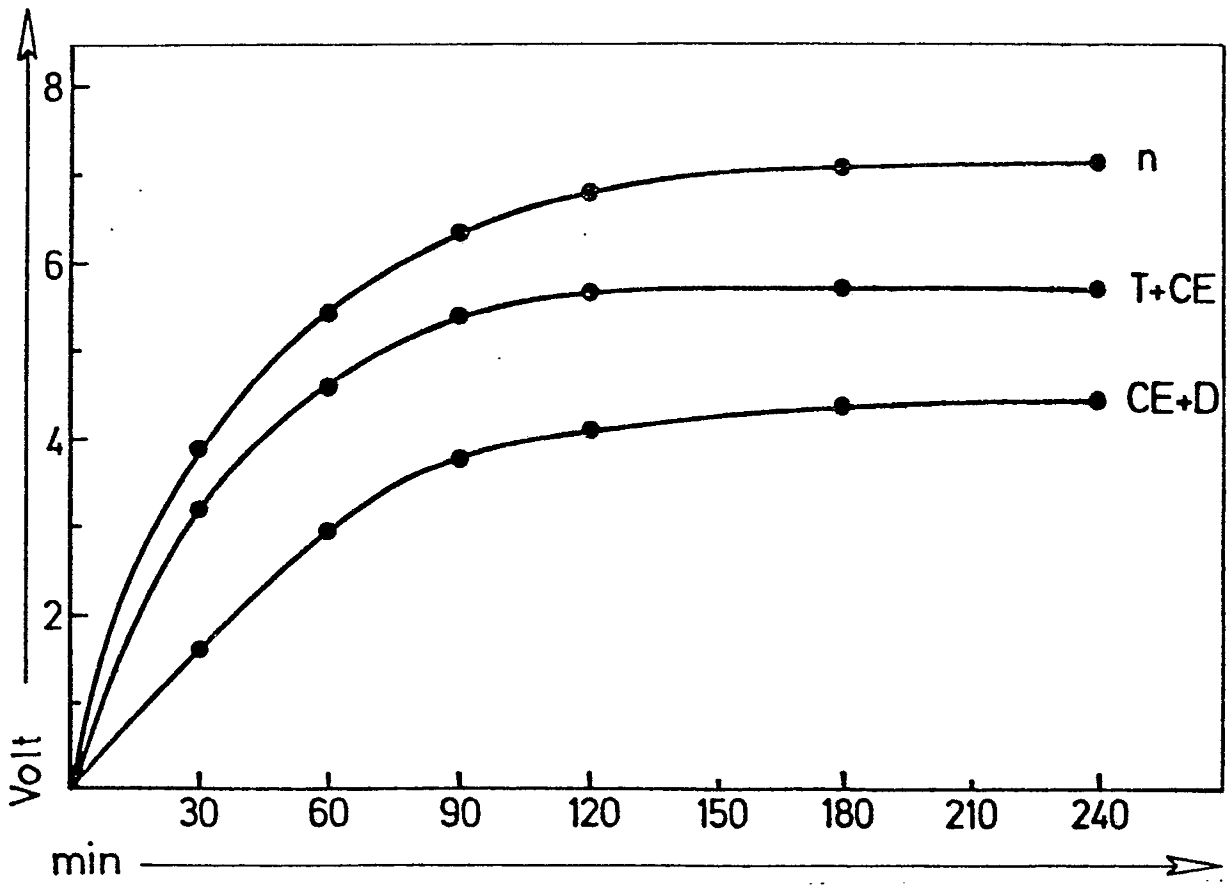

FIG. 5

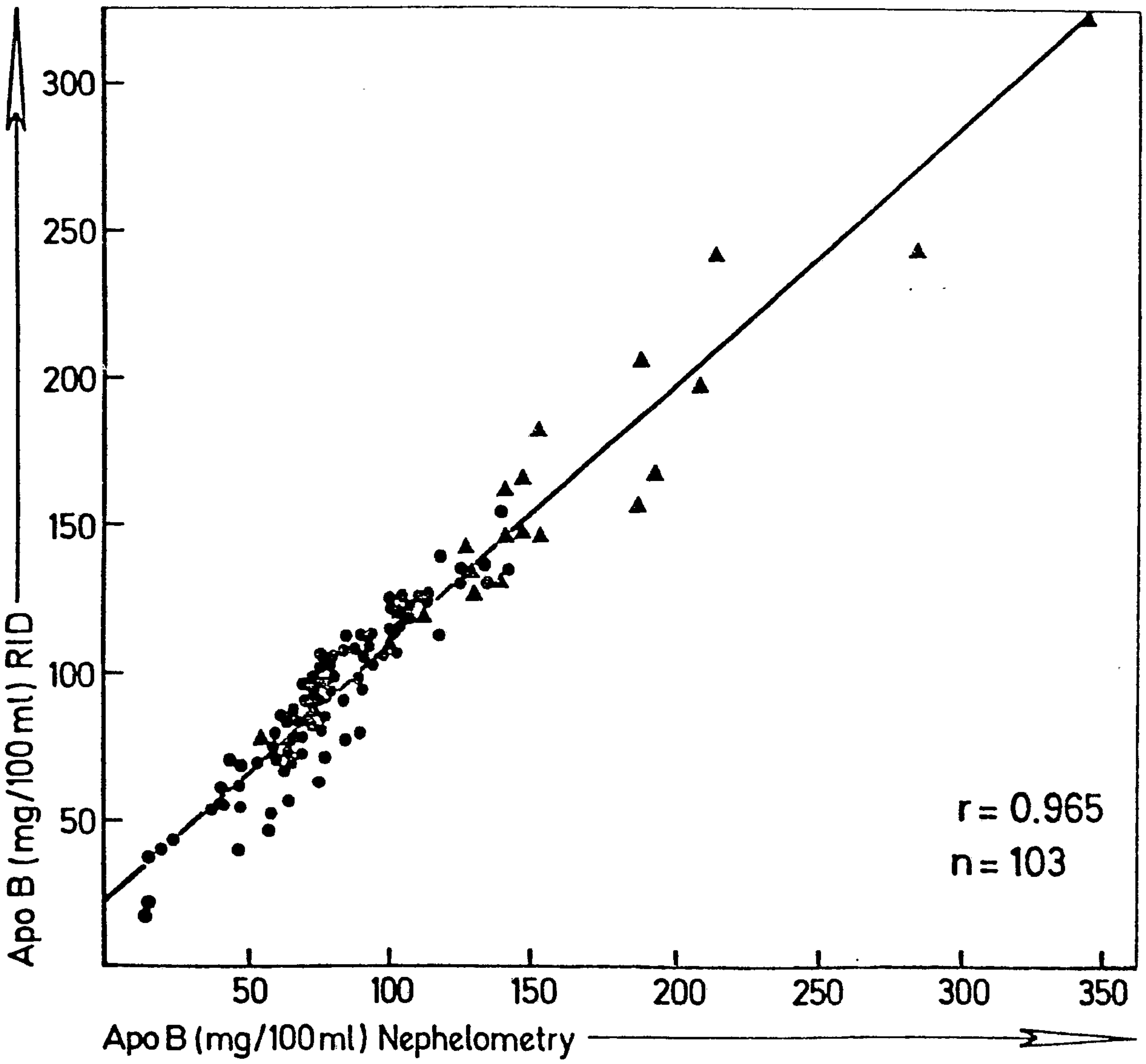
300
250
200
150
100
50
Apo B (mg/100 ml) RID
50
100
150
200
250
300
350
Apo B (mg/100 ml) Nephelometry
r = 0.965
n = 103

FIG. 6

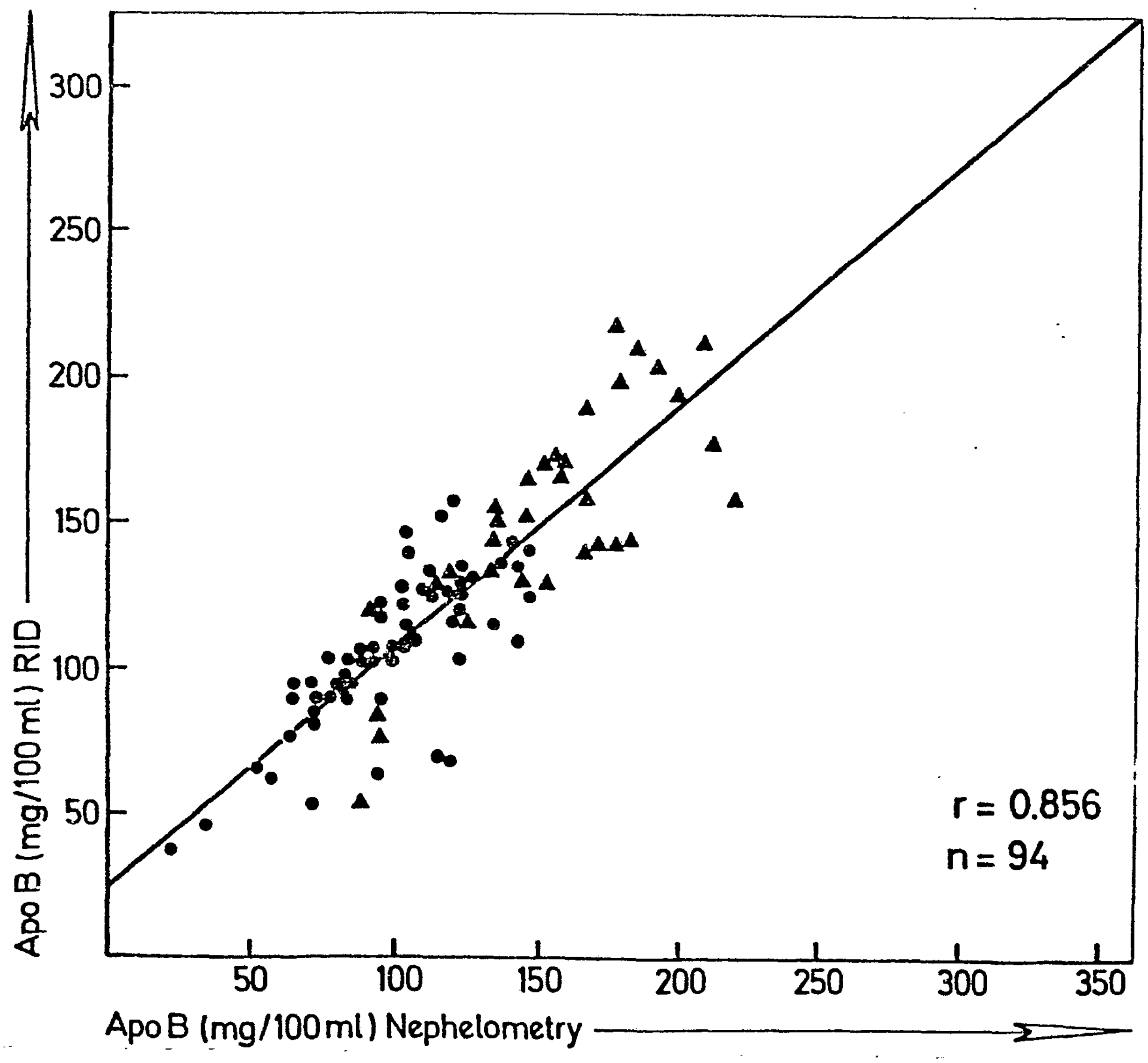
300
250
200
150
100
50
Apo B (mg/100 ml) RID
50
100
150
200
250
300
350
Apo B (mg/100 ml) Nephelometry
r = 0.856
n = 94